# EUROPEAN PATENT SPECIFICATION

(11) **EP 2 254 470 B1**
(45) Date of publication and mention of the grant of the patent: **05.10.2016**
(21) Application number: 09712781.5
(22) Date of filing: 20.02.2009
(51) Int. Cl.: A61B 5/145, C12Q 1/44, C12Q 1/34

(54) **PARAOXONASE 1 ENZYMATIC ACTIVITY, A RISK PREDICTOR FOR MAJOR ADVERSE CARDIOVASCULAR EVENTS**
PARAOXONASE 1 ENZYMATISCHE AKTIVITÄT, EIN RISIKOPRÄDIKTOR FÜR SCHWERE UNERWÜNSCHTE KARDIOVASKULÄRE EREIGNISSE
ACTIVITÉ ENZYMATIQUE DE LA PARAOXONASE 1, PRÉDICTEUR DE RISQUE POUR LES ÉVÉNEMENTS CARDIOVASCULAIRES MAJEURS

(30) Priority: 20.02.2008 US 30001 P; 14.03.2008 US 48471
(43) Date of publication of application: 01.12.2010
(73) Proprietor: THE CLEVELAND CLINIC FOUNDATION, Cleveland, OH 44195 (US)
(72) Inventor: HAZEN, Stanley, L., Pepper Pike OH 44124 (US); NICHOLLS, Stephen, James, Cleveland Heights OH 44118 (US)
(74) Representative: Tollervey, Rebecca Marie
(86) International application number: PCT/US2009/034651
(87) International publication number: WO 2009/105622

(56) References cited:
- US-A1- 2007 224 657
- US-A1- 2008 009 020
- VAN HIMBERGEN T M ET AL: "Paraoxonase (PON1) and the risk for coronary heart disease and myocardial infarction in a general population of Dutch women [online 27.12.2007]" ATHEROSCLEROSIS, ELSEVIER IRELAND LTD, IE, vol. 199, no. 2, 27 December 2007 (2007-12-27), pages 408-414, XP023440120 ISSN: 0021-9150 DOI: DOI:10.1016/J.ATHEROSCLEROSIS.2007.11.018 [retrieved on 2007-12-27]
- VAN DER A DAPHNE L ET AL: "Non-transferrin-bound iron and risk of coronary heart disease in postmenopausal women." CIRCULATION 25 APR 2006 LNKD- PUBMED:16618820, vol. 113, no. 16, 25 April 2006 (2006-04-25), pages 1942-1949, XP002623098 ISSN: 1524-4539
- KIM ET AL: "Decreased paraoxonase-1 activity is a risk factor for ischemic stroke in Koreans" BIOCHEMICAL AND BIOPHYSICAL RESEARCH COMMUNICATIONS, ACADEMIC PRESS INC. ORLANDO, FL, US, vol. 364, no. 1, 22 October 2007 (2007-10-22), pages 157-162, XP022308534 ISSN: 0006-291X DOI: DOI:10.1016/J.BBRC.2007.09.119
- MACKNESS ET AL.: 'Low Paraoxonase Activity Predicts Coronary Events in the Caerphilly Study.' CIRCULATION vol. 107, 10 June 2003, pages 2775 - 2779
- GAIDUKOV ET AL.: 'The Development of Human Sera Tests for HDL-bound Serum PON1 and its Lipolactonase Activity.' JOURNAL OF LIPID RESEARCH vol. 48, 13 April 2007, pages 1637 - 1646
- VAN HIMBERGEN T M ET AL: "Paraoxonase (PON1) and the risk for coronary heart disease and myocardial infarction in a general population of Dutch women", ATHEROSCLEROSIS, ELSEVIER IRELAND LTD, IE, vol. 199, no. 2, 1 August 2008 (2008-08-01), pages 408-414, XP023440120, ISSN: 0021-9150, DOI: 10.1016/J.ATHEROSCLEROSIS.2007.11.018 [retrieved on 2007-12-27]

## Description

### GOVERNMENT INTEREST

### BACKGROUND

The present invention relates to the field of cardiovascular disease. More specifically, it relates to methods which can be used to determine whether an individual or test subject, more particularly a human subject, without significant angiographic evidence of coronary artery disease is at a low risk or high risk of experiencing a first time major adverse cardiovascular event near term.

Cardiovascular disease (CVD) is the general term for heart and blood vessel diseases, including atherosclerosis, coronary heart disease, cerebrovascular disease, and peripheral vascular disease. Major adverse cardiovascular events are acute manifestations of CVD and include myocardial infarction, stroke, angina pectoris, transient ischemic attacks, and congestive heart failure. CVD accounts for one in every two deaths in the United States and is the number one killer disease. Thus, prevention of cardiovascular disease is an area of major public health importance.

Currently, several risk factors are used by members of the medical profession to assess an individual's risk of developing or having CVD and to identify individuals at high risk. Major risk factors for cardiovascular disease include hypertension, family history of premature CVD, smoking, high total cholesterol, low HDL cholesterol, and diabetes. The major risk factors for CVD are additive, and are typically used together by physicians in a risk prediction algorithm to target those individuals who are most likely to benefit from treatment for CVD. These algorithms achieve a high sensitivity and specificity for predicting 15% risk of CVD within 10 years. However, the ability of the present algorithms to predict a higher probability of developing CVD and/or experiencing a major adverse cardiac event is limited. Among those individuals with none of the current risk factors, the 10-year risk for developing CVD is still about 2%. In addition, a large number major adverse cardiovascular events occur in individuals with apparently low to moderate risk profiles, as determined using currently known risk factors or in individuals who have no clinical evidence of CVD, no history of CVD and/or a normal cardiac catherization. Thus, there is a need for methods of determining the risk for experiencing for the first time a major adverse cardiac event near term, i.e., within three years, in a broader spectrum of individuals, particularly in those individuals who have little to no clinical evidence of CVD.

### SUMMARY OF THE INVENTION

The present invention provides new methods for assessing the risk an individual without significant evidence of cardiovascular disease (CVD) has of experiencing a major adverse cardiac event near term. As used herein, "significant evidence of cardiovascular disease" means that the individual, subject, or patient has no history of CVD and/or less than 50% stenosis of all major coronary vessels. As used herein the term "major adverse cardiac event" includes, but is not limited to, heart attack, a myocardial infarction, stroke, and death. As used herein, the term "near term" means within three years. Thus, subjects who are at near term risk may be at risk of experiencing a major adverse cardiac event within the following day, 3 months, 6 months or one year after being tested. The present tests are especially useful for identifying those individuals who are at high risk and thus, in need of highly aggressive CVD therapies as well as those individuals who require no therapies targeted at preventing such events.

In one aspect, the present methods comprise determining the level of enzymatic activity of the protein paraoxonase 1 (PON1) in a blood, serum, or plasma sample obtained from an individual or test subject who has no history of CVD and/or a "normal" coronary angiogram obtained during cardiac catherization or other imaging technique such as multi-slice cardiac CT or MRI. The level of PON1 enzymatic activity in the bodily sample from the test subject is then compared to a control value or representative value that is derived from measurements of PON 1 enzymatic activity in comparable bodily samples obtained from the general population or a select population of human subjects (e.g. subjects who have not experienced a major adverse cardiac event within 3 years of providing the test sample), or to a baseline value. Test subjects whose blood levels of PON1 activity are lower than the control value, representative value or baseline value are at greater risk of experiencing a first time major adverse cardiac event, (e.g. a myocardial infarction, stroke, etc.) than individuals whose blood PON1 enzymatic activity levels are at or higher than the control value. Moreover, the extent of the difference between the test subjects PON1 enzymatic activity levels and control value is also useful for characterizing the extent of the risk and thereby, determining which individuals would most greatly benefit from certain therapies.

In another aspect, the present invention provides methods of determining whether a subject who has experienced a myocardial infarction or other major adverse cardiac event is at risk of experiencing a recurrent major cardiovascular event, e.g. reinfarction, near term.

### BRIEF DESCRIPTION OF THE DRAWINGS

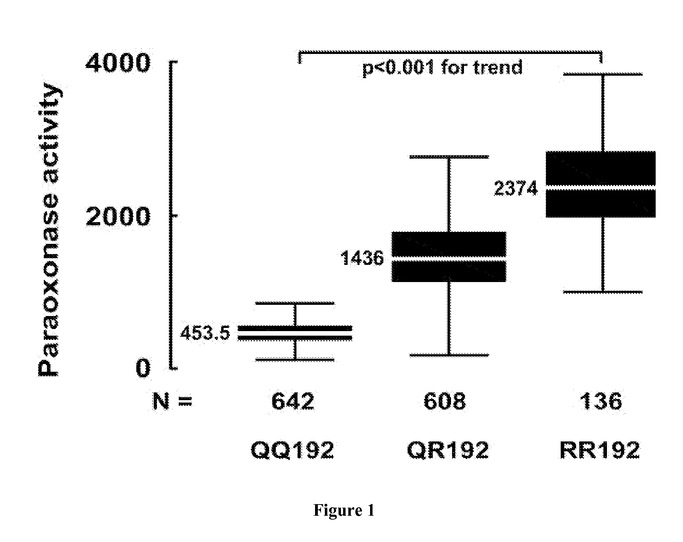

Figure 1 is a box whisker plot showing paraoxonase activity according to PON1 Q₁₉₂R genotypes. Q192R: glutamine to arginine mutation at position 192 of the protein sequence; QQ192: wild type (subjects without the mutation); QR192: heterozygotes (subjects with one copy of each allele); RR192: mutant homozygous (subjects with the mutation or variant alleles); N: number of subjects with each genotype; paraoxonase activity: expressed in nmols/min/ml of serum; 1 unit of paraoxonase activity is the amount of para-nitrophenol produced in nmols per minute per milliliter of serum; lower end of whisker = 10^{th} percentile; upper end of whisker = 90^{th} percentile; lower end of box = 25^{th} percentile or first quartile; upper end of box = 75^{th} percentile or the third quartile; center white solid line = median or 50^{th} percentile; box: interquartile range; whiskers: range of activity levels (excluding outliers).

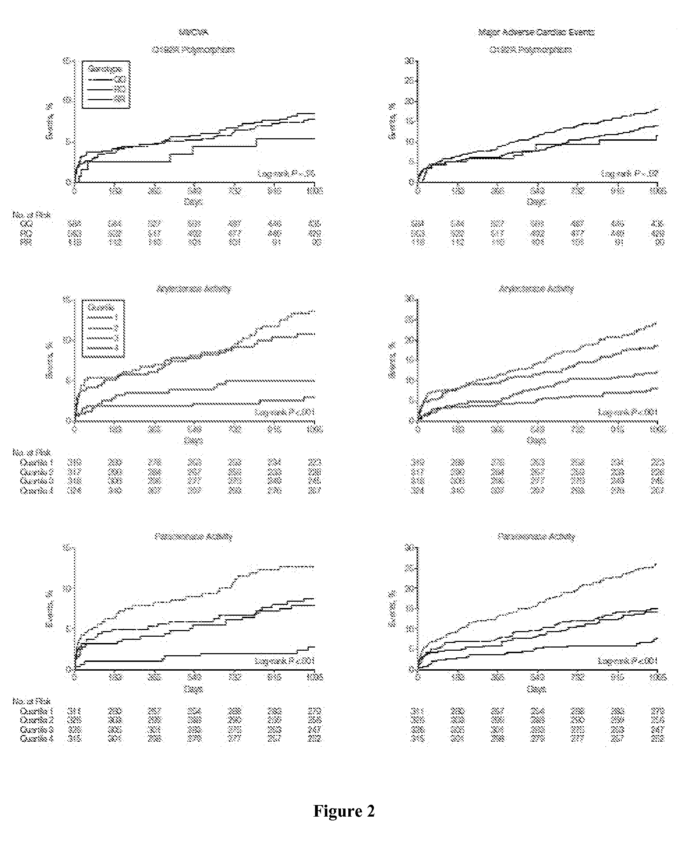

Figure 2 provides Kaplan Meier survival curves showing the relationship between functional genetic and biochemical indices of PON1 activity and incident cardiovascular risk. The Q₁₉₂R genotypes: RR₁₉₂ (mutant homozygous); QR_{192:} (heterozygous); QQ₁₉₂: (wild type). In the top panels, log-rank p values are shown for the at risk genotype QQ₁₉₂ vs. RR₁₉₂ + QR₁₉₂. PON1 activity (paraoxonase and arylesterase) were categorized into quartiles. Q1: lowest activity quartile; Q4: highest activity quartile. In the bottom panels, log-rank p values across activity quartiles are shown. Days (number of days from enrollment to first cardiac event). Event rate calculated at 6 months interval.

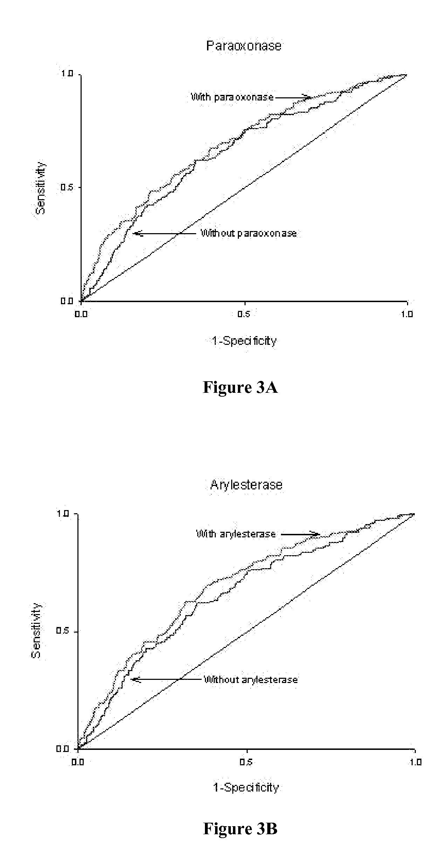

Figure 3 provides receiver operating characteristic curves showing the prognostic utility of systemic PON1 activity measures. Receiver operating characteristic curves for prediction of certain major adverse cardiac events (MACE) over incident 3 year interval using traditional cardiovascular risk factors (age, gender,race, h/o diabetes mellitus, smoking, systolic and diastolic blood pressure, high density lipoprotein, low-density lipoprotein, triglycerides, log body mass index, log c-reactive protein and medications (statins, aspirin)) with and without addition of systemic PON1 activity measures. (i) Paraoxonase activity; (ii) Arylesterase activity. Without biomarker (risk factors only): (blue line); With biomarker: (red line). A: (+log paraoxonase); B: (+log arylesterase).

### DETAILED DESCRIPTION OF THE INVENTION

The present invention will now be described by reference to more detailed embodiments, with occasional reference to the accompanying drawings. This invention may, however, be embodied in different forms and should not be construed as limited to the embodiments set forth herein. Rather these embodiments are provided so that this disclosure will be thorough and complete, and will convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for describing particular embodiments only and is not intended to be limiting of the invention. As used in the description of the invention and the appended claims, the singular forms "a," "an," and "the" are intended to include the plural forms as well, unless the context clearly indicates otherwise.

Unless otherwise indicated, all numbers expressing quantities of ingredients, properties such as molecular weight, reaction conditions, and so forth as used in the specification and claims are to be understood as being modified in all instances by the term "about." Accordingly, unless otherwise indicated, the numerical properties set forth in the following specification and claims are approximations that may vary depending on the desired properties sought to be obtained in embodiments of the present invention. Notwithstanding that the numerical ranges and parameters setting forth the broad scope of the invention are approximations, the numerical values set forth in the specific examples are reported as precisely as possible. Any numerical values; however, inherently contain certain errors necessarily resulting from error found in their respective measurements.

Provided herein are methods of assessing the risk of experiencing a major adverse cardiac event near term in a subject who has no history of CVD and/or a "normal" cardiac catherization. The methods comprise determining the levels of PON1 enzymatic activity in a blood, serum, or plasma sample from the subject and are based on applicants' discovery that subjects who have less than 50% stenosis of their major coronary arteries and whose systemic levels of PON1 enzymatic activity are low relative to a control or representative value are at greater risk of experiencing a major adverse cardiac event near term, i.e. within 3 years, that subjects whose systemic enzymatic activity levels of PON1 are high.

PON1 enzymatic activity in the subject's blood, serum, or plasma sample can be determined using a number of different substrates. Thus, in one embodiment PON1 enzymatic activity can be determined by measuring arylesterase activity (as described below) in the blood, serum, or plasma sample obtained from the test subject. In another embodiment PON1 enzymatic activity can be determined by measuring paraoxonase activity (as described below) in the blood, serum, or plasma sample obtained from the test subject. The level of PON1 activity in the body fluid can be determined by measuring the PON1 enzymatic activity in the body fluid and normalizing this value to obtain the PON1 activity or mass per ml of blood, per ml of serum, per ml of plasma, or per weight, e.g. mg of total blood protein, etc.

### Preparation of Bodily Sample

Suitable biological samples include but are not limited to whole blood samples, samples of blood fractions, including but not limited to serum and plasma. The sample may be fresh blood or stored blood (e.g. in a blood bank) or blood fractions. The sample may be a blood sample expressly obtained for the assays of this invention or a blood sample obtained for another purpose which can be sub-sampled for the assays of this invention.

In one embodiment, the biological sample is whole blood. Whole blood may be obtained from the subject using standard clinical procedures. In another embodiment, the biological sample is plasma. Plasma may be obtained from whole blood samples by centrifugation of anti-coagulated blood containing anti-coagulants such as heparin. However, it is preferred that such samples do not contain a metal ion chelator such as EDTA since these types of compounds can inhibit PON1 enzymatic activity. Such process provides a buffy coat of white cell components and a supernatant of the plasma. In another embodiment, the biological sample is serum. Serum may be obtained by centrifugation of whole blood samples that have been collected in tubes that are free of anti-coagulant. The blood is permitted to clot prior to centrifugation. The yellowish-reddish fluid that is obtained by centrifugation is the serum.

The sample may be pretreated as necessary by dilution in an appropriate buffer solution, heparinized, concentrated if desired, or fractionated by any number of methods including but not limited to ultracentrifugation, fractionation by fast performance liquid chromatography (FPLC), or precipitation of apolipoprotein B containing proteins with dextran sulfate or other methods. Any of a number of standard aqueous buffer solutions, employing one of a variety of buffers, such as phosphate, Tris, or the like, at physiological pH can be used.

### Medical Procedures to Provide Clinical Evidence of CVD

Medical procedures for determining whether a human subject has clinical evidence of cardiovascular disease include, but are not limited to, coronary angiography, coronary intravascular ultrasound (IVUS), stress testing (with and without imaging), assessment of carotid intimal medial thickening, carotid ultrasound studies with or without implementation of techniques of virtual histology, coronary artery electron beam computer tomography (EBTC), cardiac computerized tomography (CT) scan, CT angiography, cardiac magnetic resonance imaging (MRI), and magnetic resonance angiography (MRA). Methods for determining % of stenosis in a subject's coronary arteries include coronary angiography, coronary intravascular ultrasound (IVUS), cardiac computerized tomography (CT) scan, CT angiography, cardiac magnetic resonance imaging (MRI), and magnetic resonance angiography (MRA.)

### Control/Representative Value

The level of PON1 activity in the bodily sample obtained from the test subject is compared to a control value. The control value is based upon the levels of PON1 activity in comparable samples obtained from the general population or from a select population of human subjects. For example the control value may be derived from blood, serum, and/or plasma samples obtained from "apparently healthy" individuals who have not previously had any signs or symptoms indicating the presence of atherosclerosis, such as angina pectoris, history of an acute adverse cardiovascular event such as a myocardial infarction or stroke, evidence of atherosclerosis by diagnostic imaging methods including, but not limited to coronary angiography. In certain embodiments, the control value may be derived from subjects who have not experienced a major adverse cardiac event within a predetermined period of time, e.g. one to three years, after providing the control sample. In another example, the control value can be derived from an apparently healthy nonsmoker population. "Nonsmoker", as used herein, means an individual who, at the time of the evaluation, is not a smoker. This includes individuals who have never smoked as well as individuals who in the past have smoked but have not smoked in the previous 12 months. An apparently healthy, nonsmoker population may have a different normal range of PON1 enzymatic activity than will a smoking population. Accordingly, the control values selected may take into account the category into which the test subject falls. Appropriate categories can be selected with no more than routine experimentation by those of ordinary skill in the art.

In certain embodiments, the control value can be a baseline value derived by measuring systemic PON1 levels in the subject himself at an earlier time. Thus, the present method can be used to monitor over time the subject's risk of experiencing a major adverse cardiac event.

The control value can take a variety of forms. The control value can be a single cut-off value, such as a median or mean. The control value can be established based upon comparative groups such as where the risk in one defined group is double the risk in another defined group. The control can be a range, for example, where the general population is divided equally (or unequally) into groups, such as a low risk group, a medium risk group and a high-risk group, or into tertiles or quadrants, the lowest tertile or quadrant being individuals with substantially altered risk compared to individuals in the highest tertile or quadrant. In the case of paraoxonase enzymatic activity, which is inversely correlated with cardiac risks, subjects in the lowest tertile or quartile of paraoxonase enzymatic activity have the highest risk, and subjects in the highest tertile or quartile of paraoxonase enzymatic activity have the lowest risk.

Control values of PON1 enzymatic activity, such as for example, mean levels, median levels, or "cut-off" levels, are established by assaying a large sample of individuals in the general population or the select population and using a statistical model such as the predictive value method for selecting a positivity criterion or receiver operator characteristic curve that defines optimum specificity (highest true negative rate) and sensitivity (highest true positive rate) as described in Knapp, R.G., and Miller, M.C. (1992). Clinical Epidemiology and Biostatistics. William and Wilkins, Harual Publishing Co. Malvern, PA, which is specifically incorporated herein by reference. A "cutoff" value can be determined for each risk predictor that is assayed. The standardized method that was used in Example 1 below employs the guaiacol oxidation assay as described in Klebanoff, S.J., Waltersdorph, A.N. and Rosen, H. 1984. "Antimicrobial activity of myeloperoxidase". Methods in Enzymology. 105: 399-403).

The levels of PON1 enzymatic activity in the individual's bodily sample may be compared to a single control value or to a range of control values. If the level of PON1 enzymatic activity in the test subject's bodily sample is lower than the control value or range of control values, the test subject is at greater risk of experiencing a first time major cardiac event near term, i.e. within the ensuing three years than individuals with levels comparable to or above the control value or control range of values The extent of the difference between the test subject's risk predictor levels and control value is also useful for characterizing the extent of the risk and thereby, determining which individuals would most greatly benefit from certain aggressive therapies. In those cases, wherein the control value ranges are divided into a plurality of groups, such as the control value ranges for individuals at high risk, average risk, and low risk, the comparison involves determining into which group the test subject's level of the relevant risk predictor falls.

The present tests are useful for determining if and when certain CVD therapies, including therapeutic agents, which are targeted at preventing major adverse cardiac events should and should not be prescribed for a patient. For example, individuals with values of PON1 enzymatic activity below a certain cutoff value, or that arc in the lower tertile or quartile of a "normal range," could be identified as those in need of more aggressive intervention.

The present methods are especially useful for identifying individuals at increased risk of experiencing a first time major cardiovascular event who might otherwise not have been identified by existing screening protocols/methods.

The present application also describes methods of determining the near term risk for requiring revascularization as defined by angioplasty, stenting and coronary artery bypass grafting (CABG) in a subject who has no significant evidence of coronary artery disease. The method comprises determining the levels of PON1 enzymatic activity in the subject's blood, serum, or plasma. A subject who has no known history or significant evidence of coronary artery disease and whose blood, serum, and/or plasma levels of PON1 enzymatic activity are low relative to a control value based on levels of PON1 enzymatic activity in blood, serum, or plasma samples from the general population or a select population is a greater risk of requiring revascularization than a subject whose systemic levels of PON1 enzymatic activity are high relative to the control value.

In another aspect, the present invention provides methods of determining whether a subject who has experienced a myocardial infarction or other major adverse cardiac event is at risk of experiencing a recurrent major cardiovascular event, e.g. reinfarction, near term. The method comprises determining the levels of PON1 enzymatic activity in the subject's blood, serum, or plasma. A subject whose blood, serum, and/or plasma levels of PON1 enzymatic activity are low relative to a control value based on levels of PON1 enzymatic activity in blood, serum, or plasma samples from the general population or a select population is a greater risk of experiencing recurrent or subsequent major adverse cardiac events than a subject whose systemic levels of PON1 enzymatic activity are high relative to the control value.

The following examples of methods are for illustration only and are not intended to limit the scope of the invention.

### EXAMPLES

### Study design and sample collection

PON1 activity and functional polymorphisms were determined in serum and DNA samples of 1399 sequential consenting patients who participated in the GeneBank study between September 2002 and November 2003. GeneBank is a single site (Cleveland Clinic) sample repository generated from patients undergoing elective diagnostic coronary angiography with extensive clinical and laboratory characterization and longitudinal observation. For systemic measures of oxidative stress, whole blood collected in EDTA tubes was immediately spun, plasma and buffy coat isolated, and plasma stored under argon atmosphere with antioxidant cocktail supplement as previously described ²⁰. Serum for PON1 activity measures was obtained from serum separator tubes after 30-60 minutes clotting time at room temperature. All specimens were stored at -80°C until time of analysis. Patients were followed on an annual basis for adjudicated incident major adverse cardiac events and mortality until December 14, 2006. The GeneBank study was approved by the Institutional Review Board of the Cleveland Clinic. All patients provided written informed consent prior to being enrolled in the study.

### Clinical diagnosis and definition of outcomes

Information regarding demographics, medical history and medication use was obtained by patient interviews and confirmed by chart reviews. Race information utilized in analyses was pre-specified prior to the study and was based on self-report. All clinical outcomes data were verified by source documentation. Mortality was assessed using the Social Security Death Index ²². CVD was defined by the presence of coronary artery disease (CAD) or peripheral arterial disease (PAD). CAD included adjudicated diagnoses of stable or unstable angina, myocardial infarction (MI) (adjudicated definition based on defined electrocardiographic changes or elevated cardiac enzymes) or angiographic evidence of ≥ 50% stenosis of one or more epicardial vessels. PAD was defined as the presence of any extracoronary atherosclerosis, and included obstructive disease, including a history of intermittent claudication, or amaurosis fugax, or history of a cerebrovascular accident (CVA), or evidence of either arterial stenosis or aneurismal disease in the limbs or thoracic or abdominal aorta on doppler ultrasound. Prospective cardiovascular risk was assessed by the incidence of major adverse CVD events (MACE), which included non-fatal and fatal MI, non-fatal and fatal stroke or all cause mortality. Non-fatal events were defined as patients with MI or stroke who survived at least 48 hours following the onset of symptoms. We also assessed the risk of true incidence or the first cardiovascular event in subjects without a history of CVD at enrollment (baseline) and the risk for recurrent cardiovascular events in subjects with established diagnosis of CVD at baseline, according to levels of PON1 activity.

### Determination of PON1 activity

Serum arylesterase and paraoxonase activities were independently measured by UV spectrophotometry in a 96 well plate format (Spectramax 384 Plus, Molecular Devices, Sunnyvale CA), using phenyl acetate or paraoxon (Sigma-aldrich, St. Louis, MO) as substrates, respectively ²³. Briefly, for arylesterase assays, initial hydrolysis rates were determined at 270 nm in 50-fold diluted serum (final) in reactions mixtures comprised of 3.4 mM phenylacetate, 9mM Tris/HCl, pH 8, and 0.9 mM CaCl₂ at 24°C. An extinction coefficient (at 270 nm) of 1310 M⁻¹, cm⁻¹ was used for calculating units of arylesterase activity, which are expressed as the amount of phenyl acetate hydrolyzed in µmols / min per ml of serum. For paraoxonase activity assays, rate of generation of para-nitrophenol was determined at 405 nm in 40-fold diluted serum (final) in reaction mixtures comprised of 1.5mM paraoxon, 10mM Tris/HCl, pH 8, 1M NaCl and 2mM CaCl₂ at 24°C. An extinction coefficient (at 405 nm) of 17,000 M⁻¹, cm⁻¹ was used for calculating units of paraoxonase activity, which are expressed as the amount of p-nitrophenol produced in nmols / min per ml of serum. Both paraoxonase and arylesterase assays for each sample were performed in duplicates with average measurements of enzyme activity for each sample calculated. Each 96-well plate included blank samples to monitor spontaneous hydrolysis of substrates, and aliquots of serum samples of three pooled calibrators (low, mid and high levels) with known activity levels to ensure assay quality of each plate based upon established acceptability criterion. The intra-assay and inter-assay coefficient of variance (CV%) for performance of arylesterase was 1.2% and 3.9%, respectively, and the intra-assay and inter-assay CV% for performance of paraoxonase activity assays was 2.0% and 5.6%, respectively, on 20 replicates performed on 10 different days.

To establish normal ranges, serum arylesterase and paraoxonase activities were also determined on 100 apparently healthy volunteers (50 males and 50 females) ≥55 years (mean 64±4 years) responding to local advertisements. Systemic arylesterase activity in this healthy middle aged population ranged from 169.3 to 814.0, with median (IQR) levels of 605.8 (517.6 - 666.8) µmols/min/ml serum. Systemic paraoxonase activity in this healthy middle aged population ranged from 436.4 to 4025, with median (IQR) levels of 1264 (647.0 - 1865) nmols/min/ml of serum.

### Genotyping

Of the 1399 subjects, 1386 DNA samples from the GeneBank cohort were available for genotyping for the presence of PON1 Q₁₉₂R polymorphism (SNP ref ID: rs662). Primers for amplifying the sequences containing the single nucleotide polymorphism (SNP) were designed using Primer 3 (www.genome.wi.mit.edu/cgi-bin/primer/primer3_www.cgi). Sense and antisense probes used for fluorescence polarization-single base extension (FP-SBE) detection of SNP were designed using Primer Premier. Each genotyping reaction consisted of a singleplex polymerase chain reaction (PCR) followed by a single base pair extension reaction using either the sense or antisense probe and dideoxynucleotides labeled with TAMRA or R110 for the alternative alleles. The fluorescence signal was detected by fluorescence polarization using the analyst HT (Molecular Devices, Sunnyvale, CA) and genotypes were determined based on the plot of TAMRA versus R110 signal values.

### Mass spectrometry assays

Plasma levels of structurally specific species of hydroxy-eicosatetraenoic acids (HETEs), hydroxy-octadecadienoic acids (HODEs), the 8-isoprostane prostaglandin F_{2α} (8-iso PGF_{2α}) and their precursor fatty acids (arachidonic or linoleic acids) were quantified in samples of subjects using stable isotope dilution high performance liquid chromatography with on-line electrospray ionization tandem mass spectrometry (LC/ESI/MS/MS). PGF_{2α}-d₄ (Cayman Chemicals, Ann Arbor, MI) was used as internal standard for calibration of 8-isoPGF_{2α} and 15-HETE-d₈ (Cayman Chemicals, Ann Arbor, MI) for other oxidized fatty acids. Quantification of total plasma levels of each analyte (free plus esterified) were performed following addition of the appropriate isotopically labeled internal standards and base catalyzed hydrolysis, as previously described ²⁰ Analyses were performed on plasma specimens collected from fifty age, gender and race matched subjects of each genotype (QQ₁₉₂, QR₁₉₂, and RR₁₉₂) randomly selected amongst the 1386 patients. To avoid bias, fifty subjects harboring the genotype of the lowest frequency in our cohort (RR₁₉₂) were initially randomly selected amongst the 136 subjects who carried that genotype [50% male; all Caucasians and average age (63±2) years]. An additional 50 subjects from each of the QQ₁₉₂ and QR₁₉₂ cohorts, respectively, matched with the QQ₁₉₂ subjects for age, race and gender were also selected for mass spectrometry analyses.

### Statistical analyses

Clinical diagnosis, outcome definition, determination of PON1 activity, genotyping and mass spectrometry analyses were each performed by investigators who were blinded to CVD status and other measurements. Continuous variables are presented as means (±SD) or median (with interquartile ranges) for non-normally distributed data and categorical variables as numbers and percentages. Regression analysis was used for calculating the variance (R²) explained by the PON1 polymorphism and activity measures. Quartile cut-points were determined from the enzyme activity levels of all 1399 study subjects at baseline. Analysis of variance (ANOVA) or Kruskal-Wallis test (for non-normally distributed data) was used to test the difference in mean oxidized fatty acid levels according to PON1 genotype and tertiles of PON1 activity. Kaplan Meier methods were used to plot time to event curves for PON1 activity quartiles and genotypes and the log rank test was performed to assess differences between curves.

Cox proportional hazard models met assumptions of proportionality and were performed to determine if PON1 is an independent predictor of future cardiac events. All variables used in the models met the proportional hazards assumption. Unadjusted hazard ratios (HR) for clinical events was calculated with reference to the highest quartile (corresponds to lowest risk). The models were adjusted for all traditional cardiac risk factors including Framingham ATPIII risk score (including diabetes status), log CRP, BMI, and medication use (statin and aspirin). Models were created separately for paraoxonase and arylesterase. Logistic regression analysis was used to calculate adjusted odds ratios (OR) for CVD status after adjusting for the traditional risk factors and selected classes of medications (statins and aspirin) as described above. All variables used in the models met the proportional hazards assumption by testing them as time dependent covariates in the multivariate model.

Receiver operating curves were plotted to estimate C-index for MACE with and without PON1 activity as predictors ²⁴. C-index is analogous to the area under the curve but takes into account right censoring ²⁵. To evaluate the contribution of PON1 activity as a predictive marker, we calculated the concordance indices ²⁶ with and without each PON1 activity measurement in separate multivariate models that included the variables described above. The improvement in predictability with the addition of each of these well-established risk factors was assessed by the difference in the concordance indices ²⁷. The difference of the indices was bias corrected and bootstrapping was utilized to generate 95% confidence intervals. A one-sample t-test was performed to determine if the difference was equal to zero. Hazard ratios for clinical events were calculated for subjects with the PON1 Q₁₉₂R, polymorphism. Hazard ratios were adjusted after controlling for differences in the traditional cardiac risk factors and medications as above. An additive model of inheritance was also created to assess the change in risk for having one and two copies of the Q allele. The genotypes were given values of 0, 1 and 2 and entered in a Cox proportional hazards model. All statistical analyses were performed using SPSS (version 11) and verified on SAS 8.2 (SAS Inc, Cary, NC) software. All p-values are two-sided with values <0.05 considered significant.

### Results

### Clinical, laboratory and demographic characteristics

The clinical characteristics of subjects stratified according to a diagnosis of CVD at time of enrollment are summarized in Table 1. Subjects with CVD were older, with a greater prevalence of history of hyperlipidemia, diabetes and hypertension and greater use of aspirin and statins. While 66% of patients were on statins, <10% of the cohort were on fenofibrates. CVD subjects had lower levels of HDL-C and LDL-C (the latter presumably due to increased statin use) and higher levels of triglyceride and CRP. In the entire cohort 46.3% (n/N, 642/1386) harbored the QQ₁₉₂ genotype, 43.9% (n/N, 608/1386) had the QR₁₉₂ genotype, and 9.8% (n/N, 136/1386) had the RR₁₉₂ genotype. Caucasian subjects demonstrated a greater frequency of the QQ192 genotype [n/N (%), 621/1254 (49.5%) vs. 17/126 (13.5%); p<0.001] and lower frequency of the RR192 genotype [n/N (%), 92/1254 (7.4%) vs. 44/126 (34.9%); p<0.001] compared with non-Caucasian subjects. Irrespective of race, paraoxonase and arylesterase activity levels were significantly lower in subjects with CVD in comparison to non-CVD patients: median (interquartile range) 860.6 (442.6-1599) vs. 1164 (473.5-1825) nmols/min/ml, p<0.001, and 334.6 (279.5-395.8) vs. 356.9 (296-424.4) µmols/min/ml, p<0.001, for paraoxonase and arylesterase, respectively) (see Table 1 below). Similarly, systemic levels of serum paraoxonase and arylesterase activity were lower in subjects with CVD compared with healthy middle aged volunteers, 860.6 (442.6-1599) vs.1264 (647.0-1864.6) nmols/min/ml, p<0.001, and 334.6 (279.5-395.8) vs. 605.8 (517.6-666.8) µmols/min/ml, p<0.001, for paraoxonase and arylesterase, respectively. During follow up (mean 44±7 months, minimum of 3 years), 13.8% (n/N, 193/1399) of subjects experienced at least one MACE [comprised of 7.6% death (n/N, 106/1399), 5.7% non-fatal myocardial infarction (n/N, 80/1399) and 1.1% stroke (n/N, 16/1399)].

**Table 1: Sequential subjects undergoing elective diagnostic cardiac catheterization (N = 1,399)**

| Baseline Characteristics | | CVD (n = 1116) | Non-CVD (n = 283) | p value |
|---|---|---|---|---|
| Age (years) | | 65.1 ± 10.9 | 57.2 ± 11.8 | <0.001 |
| Male - n/N (%) | | 799/1116 (71.6) | 135/283 (47.7) | <0.001 |
| Caucasians - n/N (%) | | 1023/1111 (92.1) | 243/282 (86.2) | 0.002 |
| African Americans - n/N (%) | | 71/1111 (6.4) | 38/282 (13.5) | <0.001 |
| Hyperlipidemia - n/N (%) | | 940/1093 (86.0) | 168/277 (60.6) | <0.001 |
| Hypertension - n/N (%) | | 843/1100 (76.6) | 150/282 (53.2) | <0.001 |
| Diabetes Mellitus - n/N (%) | | 433/1095 (39.5) | 49/271 (18.1) | <0.001 |
| Current Smoking - n/N (%) | | 163/1116 (14.6) | 33/283 (11.7) | 0.2 |
| Statin use - n/N (%) | | 717/1088 (65.9) | 80/270 (29.6) | <0.001 |
| Aspirin use - n/N (%) | | 873/1098 (79.5) | 176/272 (64.7) | <0.001 |
| Body Mass Index (kg/m²) | | 29.7 ± 5.9 | 29.7 ± 6.3 | 0.58 |
| Systolic BP (mmHg) | | 134.3 ± 21.2 | 134.8 ± 21.3 | 0.96 |
| Diastolic BP (mmHg) | | 74.0 ± 13.2 | 75.5 ± 12.2 | 0.20 |
| HDL (mg/dL) | | 46.1 ± 13.0 | 53.9 ± 15.9 | <0.001 |
| LDL (mg/dL) | | 99.0 ± 37.5 | 112.4 ± 36.6 | <0.001 |
| Triglycerides (mg/dL) | | 138.0 (99.0 - 202.0) | 115.5 (77.0 - 173.0) | <0.001 * |
| Total Cholesterol (mg/dL) | | 178.4 ± 46.4 | 193.9 ± 43.5 | <0.001 |
| C-Reactive Protein (mg/L) | | 3.0 (1.5 -7.0) | 2.4 (1.2 - 5.9) | 0.005 * |
| Framingham ATPIII Score | | 13.3 ± 3.5 | 11.4 ± 5.0 | <0.001 |
| | | | | |
| Paraoxonase (activity unit) | | 860.6 (442.6 - 1599.0) | 1164.0 (473.5 - 1825.0) | 0.001 * |
| Arylesterase (activity unit) | | 334.6 (279.5 - 395.8) | 356.9 (296.0 - 424.4) | <0.001 |
| Prevalent disease | Genotype | Yes (N=962) | No (N=405) | p value |
| | QQ - n(%) | 461 (47.9) | 169 (41.7) | 0.03 |
| CAD ** | QR - n(%) | 410 (42.6) | 191 (47.2) | 0.12 |
| | RR - n(%) | 91 (9.5) | 45 (11.1) | 0.35 |
| | | Yes (N=363) | No (N=1023) | p value |
| | QQ - n(%) | 171 (47.1) | 471 (46.0) | 0.73 |
| PAD | QR - n(%) | 164 (45.2) | 444 (43.3) | 0.56 |
| | RR - n(%) | 28 (7.7) | 108 (10.6) | 0.12 |
| Prospective events | | CVD*** | Non-CVD*** | p value |
| MI- n (KM est., 95% CI) | | 69 (7.2, 5.6-8.9) | 11 (4.4, 1.8-6.9) | 0.14 |
| MI/CVA - n (KM est. 95% CI) | | 84 (8.9,7.1-10.7) | 12 (4.7, 2.1-7.4) | 0.05 |
| Death n (KM est., 95% CI) | | 99 (10.3, 8.3-12.2) | 7 (2.9, 0.8-5.0) | <0.001 |
| MACE n (KM est., 95% CI) | | 175 (17.9, 15.5-20.3) | 18 (7.2, 4.0-10.4) | <0.001 |

| | | | | |
|---|---|---|---|---|
| * Wilcoxon rank-sum test; Median (interquartile range); all other values are presented as mean ± standard deviation. ** Of the 1399 subjects, 1386 had genotype information, and 1367 had history of CAD information. *** Event rates in the parentheses are Kaplan-Meier estimates. QQ₁₉₂, wild type; QR₁₉₂, heterozygotes; RR₁₉₂, mutant homozygous. Units of paraoxonase activity are expressed as the amount of para-nitrophenol generated in nmols / min / ml serum. Units of arylestease activity are reported as amount of phenylacetate hydrolyzed in µmols / min / ml serum. HDL: high density lipoprotein; LDL: low density lipoprotein; Current smokers: history of any tobacco product within the last 12 months; Past smokers: history of any tobacco product more than 12 months ago; hyperlipidemia: total cholesterol > 200mg/dL or treatment with antihyperlipidemic agent; h/o diabetes mellitus: fasting plasma glucose ≥125mg/dL or treatment with hypoglycemic agents; BMI: body mass index. KM estimates: Kaplan-Meier estimates. | | | | |

### PON1 genotype and paraoxonase activity

The relationships between PON1 Q₁₉₂R genotypes and activity measures throughout the entire population are summarized in (Figure 1 and Table 2 below). The PON1 Q₁₉₂R polymorphism genotypes were in Hardy-Weinberg equilibrium. Subjects with the QQ₁₉₂ genotype demonstrated significantly less paraoxonase activity than subjects with either QR₁₉₂ genotype [453.5 (360.4 - 560.3) vs. 1436 (1129-1800) units, p < 0.001] or the RR₁₉₂ genotype [median (IQR), 453.5 (360.4-560.3) vs. 2374 (1978-2851) units, p<0.001] (Table 2A and Figure 1). While 49.4% (n/N, 317/642) of subjects with wild type QQ₁₉₂ genotype were in the lowest quartile of PON1 activity, only 4.4% (n/N, 6/136) of subjects with the variant RR₁₉₂ genotype were in the lowest activity quartile. In marked contrast, 3.9% (n/N, 25/642) of subjects with the QQ₁₉₂ genotype and 83.8% (n/N, 114/136) with the RR₁₉₂ genotype were in the highest activity quartile (Table 2A). In the lowest activity quartile, which is associated with increased cardiovascular risk, 92.2% (n/N, 317/344) of study subjects harbored the QQ₁₉₂ genotype and only 1.7% (n/N, 6/344) had the RR₁₉₂ genotype. The reverse trend was observed in the highest activity quartile, which is associated with lower CVD risk, with 7.2% (n/N, 25/348) of subjects having the QQ₁₉₂ genotype and 32.8% (n/N, 114/348) with the RR₁₉₂ genotype (Table 2B). These results confirm that the PON1 Q₁₉₂R polymorphism is associated with functional changes in systemic activity measures using paraoxon as substrate. Regression analysis also confirmed that this polymorphism accounts for 58.5% (R² = 0.586, p<0.0001) of the variation in serum paraoxonase activity levels throughout the population.

**Table 2(A): Distribution of paraoxonase activity quartiles in each PON1 Q₁₉₂R genotype**

| Q₁₉₂R Genotypes | | | | |
|---|---|---|---|---|
| Paraoxonase (quartile cutpoints, nmols/min/ml) | | QQ₁₉₂ (↑ Risk) | QR₁₉₂ | RR₁₉₂ (↓ Risk) |
| | | N = 642 | N = 608 | N = 136 |
| Median (IQR) | | 453.5 (360.4 - 560.3) | 1436 (1129 - 1800) | 2374 (1978- 2851) |
| Quartile 4 (↓ risk) (>1640) | n/N (%) | 25/642 (3.9) | 209/608 (34.4) | 114/136 (83.8) |
| Quartile 3 (1640 - 899.1) | n/N (%) | 14/642 (2.2) | 321/608 (52.8) | 14/136 (10.3) |
| Quartile 2 (899 - 450) | n/N (%) | 286/642 (44.5) | 57/608 (9.4) | 2/136(1.5) |
| Quartile 1 (↑ risk) (<450) | n/N (%) | 317/642 (49.4) | 21/608 (3.5) | 6/136 (4.4) |

**Table 2(B): Distribution of PON1 Q₁₉₂R genotypes in each paraoxonase activity quartile**

| Paraoxonase quartiles (cutpoints, nmols/min/ml) | | | | | |
|---|---|---|---|---|---|
| Genotype | Q₁₉₂R | Quartile 4 (>1640) (↓ Risk) | Quartile 3 (1640-899.1) | Quartile 2 (899-450) | Quartile 1 (<450) (↑ Risk) |
| | | N = 348 | N = 349 | N = 345 | N = 344 |
| QQ₁₉₂ (↑ Risk) | n/N (%) | 25/348 (7.2) | 14/349 (4.0) | 286/345 (82.9) | 317/344 (92.2) |
| QR₁₉₂ | n/N (%) | 209/348 (60.1) | 312 /349 (92.0) | 57/345 (16.5) | 21/344 (6.1) |
| RR192 (↓ Risk) | n/N (%) | 114/348 (32.8) | 14/349 (4.1) | 2/345 (0.6) | 6/344 (1.7) |

| | | | | | |
|---|---|---|---|---|---|
| QQ₁₉₂, wild type; QR₁₉₂, heterozygous; RR₁₉₂, mutant homozygous. ↑ risk: increased risk of CVD; ↓ risk: decreased risk of CVD. In panel A - N, total number of subjects with each genotype; n, number of subjects in each quartile of indicated genotype In panel B - N, total number of subjects in each quartile; n, number of subjects of each genotype within the indicated quartile PON1 genotype, paraoxonase activity and systemic oxidant stress | | | | | |

Plasma levels of multiple structurally specific oxidized fatty acids were quantified in subjects and analyzed for their relationships with PON1 genotype (Table 3A) and paraoxonase activity (Table 3B). Subjects with the RR₁₉₂ genotype had lower levels of all measured systemic indices of oxidant stress compared with age, gender and race matched subjects possessing the QQ₁₉₂ genotype (p<0.001 for all comparisons). Consistent with these findings, serum paraoxonase activity levels were inversely correlated with multiple direct systemic indices of oxidant stress in a dose dependent fashion (Table 3B). Collectively, these results provide both genetic and biochemical support for the notion that the PON1 Q₁₉₂R variant strongly influences quantitative measures of systemic oxidant stress in humans.

**Table 3(A): Total plasma oxidized fatty acid levels according to PON1 Q₁₉₂R genotypes**

| | Q₁₉₂R Genotypes | | | |
|---|---|---|---|---|
| | QQ₁₉₂ | QR₁₉₂ | RR₁₉₂ | p-value |
| Oxidized fatty acid (pmol/ml) | n=50 Median (IQR) | n=50 Median (IQR) | n=50 Median (IQR) | |
| 5-HETE | 20.1 (14.3-24.9) | 15.5 (12.6-19.2) | 9.1 (7.6-12.8) | <0.001 |
| 8-HETE | 3.0 (2.5-4.4) | 2.5 (1.8-2.7) | 1.5 (1.1-2.1) | <0.001 |
| 9-HETE | 42.7 (30.3-63.1) | 30.1 (22.0-35.2) | 17.2 (15.1-25.3) | <0.001 |
| 11-HETE | 5.8 (4.8-7.1) | 4.5 (3.7-6.1) | 3.7 (3.3-5.1) | 0.002 |
| 12-HETE | 7.6 (5.9-10.4) | 6.1 (4.9-8.1) | 4.4 (3.3-6.4) | <0.001 |
| 15-HETE | 24.3 (20.6-30.8) | 20.2 (16.2-26.3) | 13.1 (10.5-19.5) | <0.001 |
| 9-HODE | 35.5 (30.2-43.7) | 28.8 (22.6-37.1) | 20.3 (15.5-27.9) | <0.001 |
| 13-HODE | 38.7 (30.4-49.0) | 29.6 (23.0-38.9) | 22.2 (17.6-28.3) | <0.001 |
| 8-isoPGF2α | 11.1 (4.4-15.6) | 11.5 (3.0-28.5) | 2.8 (1.1-6.7) | <0.001 |

| | | | | |
|---|---|---|---|---|
| n, number of subjects in each group; QQ₁₉₂, wild type subjects; QR₁₉₂, heterozygous subjects; RR₁₉₂, mutant homozygous subjects; HETE, hydroxyl-eicosetetraenoic acid; HODE, hydroxyl-octadecadienoic acid; 8-iso PGF2α, isoprostane 8 prostaglandin 2 alpha; IQR, interquartile range. | | | | |

**Table 3 (B): Total plasma oxidized fatty acid levels according to tertiles of serum paraoxonase activity**

| | Paraoxonase activity (nmols/min/ml) of serum | | | |
|---|---|---|---|---|
| | Tertile 1 (< 630) | Tertile 2 (630.1 - 1928) | Tertile 3 (> 1928) | p-value |
| Oxidized fatty acid (pmol/ml) | n = 50 Median (IQR) | n = 50 Median (IQR) | n = 50 Median (IQR) | |
| 5-HETE | 21.8 (16.0-25.3) | 16.1 (13.1-20.7) | 8.4 (6.0-9.7) | <0.001 |
| 8-HETE | 3.3 (2.6-4.6) | 2.5 (2.2-2.7) | 1.2 (0.9-1.7) | <0.001 |
| 9-HETE | 45.2 (35.2-66.3) | 30.4 (25.3-37.5) | 15.8 (11.5-17.9) | <0.001 |
| 11-HETE | 5.9 (5.0-8.2) | 4.6 (4.1-6.3) | 3.6 (2.5-4.1) | <0.001 |
| 12-HETE | 7.8 (6.2-11.3) | 6.4 (5.4-8.7) | 3.8 (2.7-4.8) | <0.001 |
| 15-HETE | 24.7 (21.2-30.8) | 22.0 (17.4-30.7) | 11.3 (8.4-14.8) | <0.001 |
| 9-HODE | 36.3 (30.9-45.7) | 33.0 (24.8-38.8) | 18.3 (10.1-23.1) | <0.001 |
| 13-HODE | 39.8 (31.5-52.6) | 30.5 (25.5-43.4) | 19.3 (10.1-26.0) | <0.001 |
| 8-isoPGF2α | 12.1 (4.5-28.2) | 14.2 (7.8-29.4) | 1.6 (1.0-5.1) | <0.001 |

| | | | | |
|---|---|---|---|---|
| n, number of subjects in each group; Tertile 1, lowest paraoxonase activity tertile ; Tertile 2, intermediate paraoxonase activity tertile; Tertile 3, highest paraoxonase activity tertile; HETE, hydroxyl-eicosetetraenoic acid; HODE, hydroxyl-octadecadienoic acid; 8-iso PGF2α, isoprostane 8 prostaglandin 2 alpha; IQR, interquartile range. | | | | |

### Association of the PON1 Q₁₉₂R polymorphism with prevalent CAD, PAD and cardiovascular outcomes

An increased prevalence of CAD was observed in subjects with the PON1 QQ₁₉₂ genotype [n/N (%), 461/962 (47.9) versus 169/405 (41.7), p=0.03 for CAD and non-CAD, respectively]. In contrast, subjects with RR₁₉₂ genotype showed the opposite tendency with lower prevalence of CAD (Table 1). Subjects with the PON1 QQ₁₉₂ genotype were similarly observed to possess an increased likelihood of having a history of coronary artery bypass graft surgery (p=0.033) and a tendency toward increased prior history of percutaneous coronary intervention (p=0.089) (data not shown). In contrast, subjects possessing the PON1 RR₁₉₂ genotype demonstrating opposite tendencies (diminished history of coronary revascularization, p=0.085) (data not shown). Interestingly, the PON1 QR₁₉₂ genotypes showed no observed differences in prevalent PAD within the cohort (Table 1).

Of the 1386 subjects with genotype information, follow-up data was available in 1265 subjects (QQ₁₉₂, 584; QR₁₉₂, 563; and RR₁₉₂, 118). The PON1 functional polymorphism Q₁₉₂R was significantly linked to CVD outcomes within the cohort. For example, all cardiovascular adverse outcomes monitored were observed to a significantly lesser degree in patients who carried one R allele. The Kaplan Meier estimates of all cause mortality and MACE revealed a significant trend in our study subjects. Mutant homozygous and heterozygous subjects (RR₁₉₂ and QR₁₉₂) had significantly lower event rates for all cause mortality [n/N (%), 43/681 (6.75)] as compared to subjects with QQ₁₉₂ genotype [62/584 (11.10), p = 0.006]. Similarly, the event rate for MACE over the ensuing 3 year period was lower in the RR₁₉₂ or QR₁₉₂ subjects [n/N(%), 88/681 (13.6)] versus QQ₁₉₂ subjects [102/584 (18.0), p = 0.03] (Table 4 and Fig. 2 Top panels).

**Table 4: Relationship between PON1 Q₁₉₂R genotypes and cardiovascular outcome**

| | Q₁₉₂R Genotypes | | | |
|---|---|---|---|---|
| Outcome | Statistics | RR₁₉₂ +QR₁₉₂ | QQ₁₉₂ | p-value |
| Non-fatal MI/CVA | N | 681(118+563) | 584 | |
| | n/N (KM Est) | 51/681 (7.97) | 43/584 (7.88) | 0.95 |
| | HR (95% CI) | 1.0 | 1.01 (0.65 - 1.57) | 0.96 |
| Mortality | n/N (KM Est) | 43/681 (6.75) | 62/584 (11.10) | 0.006 |
| | HR (95% CI) | 1.0 | 2.05 (1.32 - 3.18) | 0.001 |
| MACE | n/N (KM Est) | 88/681 (13.59) | 102/584 (18.04) | 0.03 |
| | HR (95% CI) | 1.0 | 1.48 (1.09 - 2.03) | 0.014 |

| | | | | |
|---|---|---|---|---|
| For all analyses the (RR₁₉₂ or QR₁₉₂) group was used as the reference group. For all analyses adjusted hazard ratio is reported. HR was calculated for each clinical endpoint by adding the risk allele Q₁₉₂ to the multivariate model that included the Framingham ATPIII risk score of individual subjects (including diabetes and smoking status), race, log CRP, BMI and use of statin and aspirin. N: number of subjects in each group; n: number of subjects with the event; HR: hazard ratio; 95% CI: ninety five percent confidence interval; KM est: Kaplan -Meier estimate (a non-parametric method that measures the exact survival time of each subject rather than grouping the survival time into intervals); mortality: all cause mortality; MACE: major adverse cardiovascular events (non-fatal MI, non-fatal CVA and all cause mortality). | | | | |

In a multivariate model comprised of age, Framingham ATPIII risk score, race, log CRP, BMI, and medication (statin and aspirin) use, the addition of the risk allele Q₁₉₂ showed no effect on incident MI and stroke over the ensuing three years following enrollment, but was significantly associated with increased likelihood of death and MACE (Table 4). Compared to subjects with either the RR₁₉₂ or QR₁₉₂ genotypes, subjects with the QQ₁₉₂ genotype demonstrated an adjusted HR (95% CI) of 2.05 (1.32-3.18) for all cause mortality (p = 0.001). Furthermore, major adverse cardiac events (MACE) were also more likely to occur over the ensuing 3 year period in subjects possessing the QQ₁₉₂ genotype as compared to subjects with either the RR₁₉₂ or QR₁₉₂ genotypes [Adjusted HR (95% CI) of 1.48 (1.09-2.03), p = 0.014) [Table 4]. In a separate set of analyses, an additive model of inheritance was created to assess the change in risk for having a copy of the Q allele. An adjusted HR (95% CI) for 3 year all-cause mortality of 1.58 (1.12-2.24), p = 0.01, was observed for having a Q allele. Furthermore, in this model the presence of a Q allele was also associated with an increased risk of having a major adverse cardiac event (MACE) over the ensuing 3 year period [HR (95% CI) 1.32 (1.04-1.69), p = 0.025].

### PON1 activity and cardiovascular disease outcomes

A higher prevalence of CVD was also observed in patients with low serum activity levels of either paraoxonase or arylesterase, even following adjustment for differences in known CVD risk factors [OR (95%CI) 1.5 (0.97-2.2, p=0.07) and 2.1 (1.4-3.1, p=0.001) for comparisons between upper vs. lower quartile of paraoxonase and arylesterase activities, respectively]. Low systemic levels of enzyme activity were significantly associated with the presence of CAD [OR (95%CI) 1.5 (1.03-2.3), p=0.03 and 2.0 (1.4-3.1), p=0.001 for paraoxonase and arylesterase, respectively] or in combination with PAD [OR (95% CI) 1.7 (1.03-2.8), p<0.01 and 1.7 (1.1-2.9), p< 0.01 for paraoxonase and arylesterase, respectively]. Event rates for all prospective cardiovascular events were significantly lower in subjects in the highest PON1 activity quartile as compared to subjects in the lowest quartile (p<0.001 for all prospective events comparisons) (Table 5 and Fig. 2 middle and bottom panels). The frequency of incident cases of non-fatal MI or stroke among subjects within the highest quartiles of PON activity was only 2.8% [n/N, 8/315] based upon paraoxonase activity, and 2.3% [n/N, 9/324] using arylesterase activity. In contrast, markedly higher rates [n/N(%), 37/311 (12.8%)] and [n/N(%), 40/319 (13.7%)] of incident non-fatal MI/stroke were observed in subjects within the lowest quartile of paraoxonase and arylesterase activity, respectively. Similarly, markedly lower frequency of three year incident all-cause mortality was observed in subjects within the highest quartiles of paraoxonase [n/N(%), 17/315 (5.4%)] and arylesterase [n/N(%), 16/324 (4.9%)] activity, compared with subjects within the lowest quartiles of paraoxonase and arylesterase activity [n/N(%), 44/311 (14.1%) and 41/319 (12.9%), respectively]. Lower event rates were also noted for MACE with 7.3% [n/N, 23/315] and 7.7% [n/N, 25/324] for study subjects in the highest quartiles of paraoxonase and arylesterase activity, respectively, as compared to the event rates for MACE in study subjects in the lowest quartiles of paraoxonase and arylesterase activity [n,N(%), 78/311 (25.1%) and 75/319 (23.5%), respectively] (Table 5 and Fig. 2 middle and bottom panels). Following multivariate analysis, serum PON1 activity measures remained independently associated with prospective risk for cardiac events (Table 5). The lowest quartile of either paraoxonase or arylesterase activity were associated with a greater incident risk for non-fatal MI or stroke [HR (95% CI) 4.4 (2.0-9.6), p<0.001 and 4.5 (2.2-9.9), p<0.001, respectively] over the follow-up interval of three years. The risk for all cause mortality was also greatest in subjects in the lowest quartile of either paraoxonase or arylesterase activity [HR (95% CI) 2.4 (1.3-4.4), p= 0.004 and 2.2 (1.2-4.2), p=0.01, respectively]. This translated to a greater incidence of MACE in subjects with the lowest quartile of paraoxonase and arylesterase activity [HR (95% CI) 3.4 (2.1-5.5), p<0.001 and 2.9 (1.8-4.7), p<0.001, respectively]. Remarkably, upon further analyses, low systemic levels of arylesterase activity were associated with a pronounced risk of having the first cardiovascular event (true incidence) among subjects without either a history of CVD or angiographic evidence of significant CAD (defined by ≥ 50% stenosis) at baseline [adjusted HR (95% CI)] [5.8 (1.2-28.6), p=0.03] (Table 6A). Increased risk of having a recurrent non-fatal MI or stroke, all cause mortality and MACE was observed with low levels of paraoxonase and arylesterase activity measurements in subjects with an established diagnosis of CVD at enrollment (Table 6B). The adjusted [HR (95% CI)] for recurrent non-fatal MI or stroke was [4.5 (1.9-11.0), p=0.001] for paraoxonase and [4.2 (1.9-9.6), p=0.001] for arylesterase between the highest and lowest activity quartiles. Similarly, subjects in the lowest activity quartile for either paraoxonase or arylesterase were more likely to have a recurrent major adverse cardiovascular event (recurrent MACE) as compared to subjects in the highest activity quartile, [3.4 (2.0-5.9)], p<0.001 and [2.4 (1.4-3.9)], p<0.001.

**Table 5: Risk of prospective cardiovascular events in relation to PON1 activity**

| Paraoxonase activity quartiles (nmols/min/ml) | | | | |
|---|---|---|---|---|
| | Quartile 4 | Quartile 3 | Quartile 2 | Quartile 1 |
| | >1640 | 1640 - 899.1 | 899 - 450 | <450 |
| | N=315 | N=325 | N=326 | N=311 |
| | | | | |
| Non-fatal MI/CVA | | | | |
| n/N (%) | 8/315 (2.5) | 23/325 (7.4) | 27/326 (8.3) | 37/311 (11.9) |
| Unadjusted HR | 1.0 | 3.0 (1.3-6.7) | 3.3 (1.5-7.3) | 5.0 (2.3-10.8) |
| Adjusted HR Mortality | 1.0 | 2.9 (1.3-6.4) | 3.1 (1.4-7.0) | 4.4 (2.0-9.6) |
| n/N (%) | 17/315 (5.4) | 21/325 (6.5) | 27/326 (8.3) | 37/311 (11.9) |
| Unadjusted HR | 1.0 | 1.2 (0.6-2.3) | 1.3 (0.7-2.5) | 2.7 (1.5-4.7) |
| Adjusted HR MACE | 1.0 | 1.1 (0.6-2.2) | 1.3 (0.7-2.6) | 2.4 (1.3-4.4) |
| n/N (%) | 23/315 (7.3) | 44/325 (13.5) | 48/326 (14.7) | 78/311 (25.1) |
| Unadjusted HR | 1.0 | 1.9 (1.2-3.2) | 2.1 (1.2-3.4) | 3.7 (2.3-5.9) |
| Adjusted HR | 1.0 | 1.9 (1.1-3.2) | 2.0 (1.2-3.4) | 3.4 (2.1-5.5) |
| | | | | |

| Arylesterase activity quartiles (µmols/min/ml) | | | | |
|---|---|---|---|---|
| | Quartile 4 | Quartile 3 | Quartile 2 | Quartile 1 |
| | >403.9 | 403.9 - 338.5 | 338.4-283 | <283 |
| | N = 324 | N = 318 | N = 316 | N = 319 |
| Non-fatal MI/CVA | | | | |
| n/N (%) | 9/324 (2.8) | 15/318 (4.7) | 32/316 (10.1) | 40/319 (12.5) |
| Unadjusted HR | 1.0 | 1.7 (0.8-4.0) | 3.8 (1.8-8.0) | 4.8 (2.3-9.9) |
| Adjusted Mortality | 1.0 | 1.5 (0.7-3.6) | 3.4 (1.6-7.2) | 4.5 (2.2-9.4) |
| n/N (%) | 16/324 (4.9) | 22/318 (6.9) | 27/316 (8.5) | 41/319 (12.9) |
| Unadjusted HR | 1.0 | 1.4 (0.7-2.7) | 1.8 (1.0-3.3) | 2.7 (1.5-5.0) |
| Adjusted HR MACE | 1.0 | 1.4 (0.7-2.8) | 1.8 (0.9-3.4) | 2.2 (1.2-4.2) |
| n/N (%) | 25/324 (7.7) | 37/318 (11.6) | 56/316 (17.7) | 75/319 (23.5) |
| Unadjusted HR | 1.0 | 1.6 (0.9-2.6) | 2.4 (1.5-3.9) | 3.3 (2.1-5.2) |
| Adjusted HR | 1.0 | 1.5 (0.9-2.5) | 2.3 (1.4-3.7) | 2.9 (1.8-4.7) |

| | | | | |
|---|---|---|---|---|
| For all analysis unadjusted and adjusted hazard ratio and 95% confidence interval are reported over a three year period. Adjusted hazard ratio was adjusted for Framingham ATPIII risk score (including diabetes status), log CRP, BMI, and medication (statin and aspirin). Q4 (highest activity) was used as the reference quartile. MI, myocardial infarction; Non-fatal MI/stroke, incident cases of non-fatal MI or non- fatal stroke; Mortality, all cause mortality; Future MACE, future major adverse cardiac events (included non-fatal and fatal MI, non-fatal and fatal stroke or all cause mortality); Units of paraoxonase activity are expressed as the amount of para-nitrophenol generated in nmols / min / ml serum. Units of arylestease activity are reported as amount of phenylacetate hydrolyzed in µmols / min / ml serum. | | | | |

**Table 6(A): Incidence of first cardiovascular event in relation to PON1 activity**

| Paraoxonase activity quartiles (nmols/min/ml) | | | | |
|---|---|---|---|---|
| | Quartile 4 | Quartile 3 | Quartile 2 | Quartile 1 |
| | >1640 | 1640 - 899.1 | 899-450 | <450 |
| | N=80 | N=61 | N=57 | N=56 |
| | | | | |
| MACE | | | | |
| n/N (%) | 4/80(5.0) | 3/61 (4.9) | 3/57 (5.3) | 8/56 (14.3) |
| Unadjusted HR | 1.0 | 1.0 (0.2-4.4) | 1.1 (0.2-4.7) | 3.0 (0.9-9.8) |
| Adjusted HR | 1.0 | 0.6 (0.1-3.1) | 1.2 (0.2-5.3) | 1.7 (0.4-6.4) |
| | | | | |

| Arylesterase activity quartiles (µmols/min/ml) | | | | |
|---|---|---|---|---|
| | Quartile 4 | Quartile 3 | Quartile 2 | Quartile 1 |
| | >403.9 | 403.9 - 338.5 | 338.4 - 283 | <283 |
| | N = 91 | N = 61 | N = 51 | N = 51 |
| MACE | | | | |
| n/N (%) | 2/91 (2.2) | 3/61 (4.9) | 3/51 (5.9) | 10/51 (19.6) |
| Unadjusted HR | 1.0 | 2.2 (0.4-13.4) | 2.7 (0.4-16.0) | 9.7 (2.1-44.1) |
| Adjusted HR | 1.0 | 2.1 (0.3-12.8) | 2.4 (0.4-14.4) | 5.8 (1.2-28.6) |

| | | | | |
|---|---|---|---|---|
| For all analysis unadjusted and adjusted hazard ratio and 95% confidence interval are reported over a three year period. Adjusted hazard ratio was adjusted for Framingham ATPIII risk score (including diabetes status), log CRP, BMI and medication (statin and aspirin). Q4 (highest activity) was used as the reference quartile. N: number of subjects in each quartile; n: number of subjects who had an event in each quartile; Units of paraoxonase activity are expressed as the amount of para-nitrophenol generated in nmols / min / ml serum. Units of arylestease activity are reported as amount of phenylacetate hydrolyzed in µmols / min / ml serum. | | | | |

**Table 6(B): Risk of recurrent cardiovascular events in relation to PON1 activity**

| Paraoxonase activity quartiles (nmols/min/ml) | | | | |
|---|---|---|---|---|
| Recurrent events | Quartile 4 | Quartile 3 | Quartile 2 | Quartile 1 |
| | >1640 | 1640 - 899.1 | 899 - 450 | <450 |
| | N= 235 | N= 265 | N= 269 | N= 255 |
| Non-fatal MI/CVA | | | | |
| n/N (%) | 6/235 (2.6) | 22/265 (8.3) | 24/269 (8.9) | 40/255 (12.5) |
| Unadjusted HR | 1.0 | 3.4 (1.4-8.3) | 3.6 (1.5-8.7) | 5.3 (2.2-12.8) |
| Adjusted HR | 1.0 | 3.2 (1.3-7.9) | 3.3 (1.3-8.1) | 4.5 (1.9-11.0) |
| Mortality | | | | |
| n/N (%) | 15/235 (6.4) | 20/265 (7.5) | 24/269 (8.9) | 40/255 (15.7) |
| Unadjusted HR | 1.0 | 1.2 (0.6-2.3) | 1.4 (0.7-2.6) | 2.6 (1.4-4.6) |
| Adjusted HR | 1.0 | 1.1 (0.6-2.3) | 1.4 (0.7-2.7) | 2.4 (1.3-4.5) |
| MACE | | | | |
| n/N (%) | 19/235 (8.1) | 41/265 (15.5) | 45/269 (16.7) | 70/255 (27.5) |
| Unadjusted HR | 1.0 | 2.0 (1.2-3.4) | 2.1 (1.2-3.6) | 3.7 (2.2-6.2) |
| Adjusted HR | 1.0 | 2.0 (1.1-3.5) | 2.1 (1.2-3.7) | 3.4 (2.0-5.9) |
| | | | | |

| Arylesterase activity quartiles (µmols/min/ml) | | | | |
|---|---|---|---|---|
| Recurrent events | Quartile 4 | Quartile 3 | Quartile 2 | Quartile 1 |
| | >403.9 | 403.9 - 338.5 | 338.4-283 | <283 |
| | N = 233 | N = 257 | N = 266 | N = 268 |
| Non-fatal MI/CVA | | | | |
| n/N (%) | 7/233 (3.0) | 12/257 (4.7) | 30/266 (11.3) | 35/268 (13.1) |
| Unadjusted HR | 1.0 | 1.6 (0.6-4.1) | 4.0 (1.8-9.1) | 4.6 (2.0-10.4) |
| Adjusted | 1.0 | 1.4 (0.5-3.6) | 3.4 (1.5-7.9) | 4.2 (1.9-9.6) |
| Mortality | | | | |
| n/N (%) | 16/233 (6.9) | 22/257 (8.6) | 26/266 (9.8) | 35/268 (13.1) |
| Unadjusted HR | 1.0 | 1.3 (0.7-2.4) | 1.5 (0.8-2.8) | 1.9 (1.1-3.5) |
| Adjusted HR | 1.0 | 1.3 (0.6-2.5) | 1.4 (0.7-2.8) | 1.7 (0.9-3.2) |
| MACE | | | | |
| n/N (%) | 23/233 (9.9) | 34/257 (13.2) | 53/266 (19.9) | 65/268 (24.3) |
| Unadjusted HR | 1.0 | 1.4 (0.8-2.4) | 2.2 (1.3-3.5) | 2.6 (1.4-3.9) |
| Adjusted HR | 1.0 | 1.3 (0.7-2.3) | 2.0 (1.2-3.3) | 2.4 (1.4-3.9) |

| | | | | |
|---|---|---|---|---|
| For all analysis unadjusted and adjusted hazard ratio and 95% confidence interval are reported over a three year period. Adjusted hazard ratio was adjusted for Framingham ATPIII risk score (including diabetes status), log CRP, BMI and medication (statin and aspirin). Q4 (highest activity) was used as the reference quartile; N: number of subjects in each quartile; n: number of subjects with a recurrent event in each quartile; Units of paraoxonase activity are expressed as the amount of para-nitrophenol generated in nmols / min / ml serum. Units of arylestease activity are reported as amount of phenylacetate hydrolyzed in µmols / min / ml serum.Prognostic value of PON1: | | | | |

Some recent reports suggest that addition of multiple non-traditional risk factors does not significantly augment the prognostic utility of traditional cardiac risk factors ²⁷. Performing similar analyses, we observed that the C-index for the outcomes non-fatal MI or stroke over a minimum three year follow-up period was 0.59 (with traditional risk factors as predictors), and significantly increased to either 0.66, p=0.007 or 0.69, p=0.001 in comparisons with traditional risk factors when including either systemic measures of paraoxonase or arylesterase activities, respectively (Figure 3). For the composite MACE as an outcome, addition of either paraoxonase or arylesterase activity to traditional risk factors also significantly increased the predictive value of the model over a mean 44 (± 7) month period. For example, the C-index of 0.67 with traditional risk factors alone increased to 0.71 (p=0.007) and 0.70 (p=0.01) for risk factors plus paraoxonase or arylesterase, respectively.

### Discussion

Oxidative stress is believed to play a pivotal role in the pathogenesis of a number of chronic inflammatory disease processes, including atherosclerosis. The failure of α-tocopherol supplementation studies with alleged antioxidant properties to prevent cardiovascular events has brought the oxidation hypothesis of atherosclerosis into question²⁹. Therefore, the search continues to identify effective strategies that promote systemic antioxidant effects in humans and to determine whether they have a beneficial influence on the rate of CVD. The present genetic and biochemical studies demonstrate that the HDL-associated protein PON1 promotes pronounced systemic anti-oxidant effects in humans, with coincident links to reduction in CAD prevalence and prospective risks for MACE. Importantly, our results demonstrate that both a subject's Q₁₉₂R genotype and serum PON1 activity are associated with both prevalent CAD and incident adverse cardiovascular events.

It has been speculated that PON1 contributes to the atheroprotective property of HDL via promotion of a systemic antioxidant effect ^{1, 30}. However, there has been no definitive in vivo evidence that PON1 promotes systemic antioxidant effects in humans. In a recent meta-analysis of 43 studies examining the relationship between PON1 and clinical outcome ¹⁸ it was concluded that considerable uncertainty remained, since published studies to date primarily involve small cohorts of subjects. Moreover, they often failed to simultaneously examine PON1 genotype, systemic activity measures and cardiovascular outcomes, and none examined systemic quantitative indices of oxidant stress. Herein, we investigated the potential relationship between PON 1 activity, genotype, systemic oxidative stress and both CAD and PAD in a large prospective cohort of patients to more fully interrogate the above mentioned relationships. We demonstrate that the PON1 Q₁₉₂R polymorphism is functional, resulting in increased enzymatic activity. In parallel we demonstrate that elevated systemic levels of multiple structurally distinct fatty acid oxidation products that are increased in both atherosclerotic plaque and plasma of subjects with CVD ^{31,32}, are associated with low systemic levels of PON1 activity and the QQ₁₉₂ genotype. Importantly, the plasma samples analyzed in the present study were collected and processed under conditions designed to prevent artificial oxidation of lipids during both storage and analysis. The finding that levels of 9-HETE, an isomer of arachidonic acid oxidation produced exclusively by free radical mediated processes, are higher in subjects with low levels of paraoxonase activity suggests that PON1 can influence oxidative events beyond the cyclooxygenase and lipoxygenase pathways. While the mechanism(s) for PON1 mediated systemic antioxidant effects remains to be determined, the present findings strongly support a role for this HDL associated protein in modulating systemic oxidant stress in humans. Moreover, the present studies suggest an important mechanistic link between PON1, systemic oxidant stress and risk for development of atherosclerotic heart disease and its acute complications.

In a recent publication amino acid position 192 of PON1 was suggested to participate in HDL binding ³³. The Q¹⁹² alloenzyme was shown to bind to the HDL particle with 3-fold lower affinity than the R₁₉₂ alloenzyme, and consequently exhibited lower stability, lipolactonase activity, and modulatory effect on macrophage cholesterol efflux. The findings of the present clinical study compliment these results, demonstrating individuals harboring the arginine (R) mutation at position 192 have higher serum levels of PON activity, lower systemic indices of systemic oxidant stress, and corresponding reductions in both prevalent CAD and prospective cardiac events.

This is the first large prospective study that comprehensively examines the genetics and biochemical activity of PON1 using dual enzyme measurements to predict prevalent disease risks, as well as prospective risk for MACE, while simultaneously also examining whether a potential mechanism for CVD associations is linked to systemic oxidant stress measures in humans. Compared to prior studies that often focused on lower risk populations ¹⁸, the present cohort is also substantially enriched with patients with both CAD and PAD, enabling us to better study the association of the PON1 Q₁₉₂R variant on coronary and extracoronary atherosclerosis after controlling for established risk factors. Even so, perhaps one of the more remarkable findings in the present study is that diminished systemic arylesterase activity levels still remained significantly associated with three year incident major adverse cardiovascular event (composite of MI, stroke or death) in the subgroup of subjects without history or clinical evidence of either CAD or PAD and without significant angiographic evidence of CAD. Furthermore, the present study also suggests a potential prognostic value of PON1 activity measurement in subjects. The addition of systemic PON1 activity measures to traditional risk factors and CRP provided a significant incremental improvement in the ability to predict clinical outcome over a three year period.

It should be noted that in recent genome wide association (GWA) studies^{34, 35}, evidence for association of the PON1 gene with CAD or MI was not observed, raising the obvious question - why? One likely explanation is that GWA studies are not ideal for assessing specific candidate genes since the panel of SNPs placed on the chip may not capture all of the genetic variation for any particular gene ³⁶. Furthermore, GWA studies will tend to identify genes with the strongest genetic effect due to the stringency in considering what is statistically significant. In this regard, a weak association with PON1 may have been observed but not reported since it did not exceed the threshold for significance based on the thousands of statistical tests performed. Lastly, one of the phenotypes we have studied, namely mortality within the cohort, was not the exact same as those studied in the GWA studies, which could also account for why an association with PON1 was not reported.

The PON1 gene is located in close proximity to two other members of the paraoxanase gene family, PON2 and PON3, which raises the possibility that the association we have observed could be due to linkage disequilibrium (LD) with a variant in either PON2 or PON3. However, an examination of the HAPMAP database for the Caucasian population (which matches the GeneBank cohort) shows that the PON1 Q₁₉₂R variant is located within one haplotype block in which the SNPs are in moderate LD with each other. Moreover, this haplotype block only covers PON1 and does not extend to the PON2 and PON3 genes. Thus, while it is possible that variants of PON2 and PON3 could also contribute to altered PON activity and CVD risk, the available HAPMAP data suggests that, in Caucasians, the association observed with the PON1 Q₁₉₂R variant is not due to LD with other variants in the adjacent PON2 and PON3 genes, and likely results from altered PON1 function. Although it is possible that other PON1 variants that are in LD with the Q₁₉₂R SNP are the causal alleles, recent studies employing recombinant PON1 mutants at the 192 position have shown that this residue is important for HDL binding ³³ and suggest that the Q₁₉₂R SNP is the causal variant underlying the clinical associations that we observe. Further studies to address this issue are required.

A number of caveats to the present study should be noted. The fact that all subjects had presented for elective diagnostic coronary angiography limits the generalization of the present findings and raises the possibility of selection bias. However, measurements of both paraoxonase and arylesterase activities in healthy volunteers are higher than both CVD and non-CVD subjects evaluated in the present study. Given that the majority of subjects were Caucasians, it also remains to be determined whether the same relationships are observed in large cohorts of other race or ethnic groups. The lower percentage of non-Caucasian subjects in our cohort (7.9%) limited our ability to analyze the mechanistic link between PON1 and global indices of systemic oxidative stress and thereby its association with CVD in subjects of other racial and ethnic backgrounds. It is also uncertain whether concomitant medical problems influenced PON1 activity; however, inclusion of traditional cardiac risk factors including age, gender, race, diabetes, hypertension, smoking, lipids, CRP and medication use (statins and aspirin) in the multivariate analyses failed to alter the results.

It also is remotely possible that the reduction in PON1 activity may result from the presence of vascular disease rather than be the direct cause of future CVD events. However, arylesterase activity levels in subjects with minimal angiographic evidence of CAD still remained independently associated with incident cardiovascular events over the ensuing three year interval following subject enrollment, consistent with an association of PON1 in early macrovascular atherosclerotic disease processes. Regardless, the current observation of a relationship between the PON1 Q₁₉₂R polymorphism and activity, oxidative stress and CVD outcomes is consistent with findings in murine models and provides evidence that PON1 protein protects against the development and propagation of CVD.

In summary, the current findings provide direct prospective evidence of an important mechanistic link between the PON1 gene and systemic activity measures with both multiple quantitative indices of oxidative stress and atherosclerotic heart disease development in humans. PON1 is almost exclusively found to be associated with HDL particles within the circulation and has been argued to promote some of the anti-inflammatory and anti-oxidant effects attributed to HDL. Thus, the present studies also provide further support for the concept that functional properties beyond the ability of HDL and its associated proteins to promote reverse cholesterol transport contribute to the overall ability of this lipoprotein to retard or prevent development of atherosclerosis. Finally, the present findings further suggest the potential utility of using PON 1 activity for evaluating whether therapeutic strategies aimed at elevating HDL are similarly elevating its associated atheroprotective proteins, such as PON1, thus bolstering the beneficial effects of the intervention.

## Claims

1. A method for assessing the risk of a subject experiencing a major adverse cardiac event within 3 years of the assessment, wherein the subject has no history of cardiovascular disease and/or less than 50% stenosis of all major coronary vessels, the method comprising:
determining the levels of paraoxonase 1 (PON1) enzymatic activity in a blood, serum, and/or plasma sample from the subject;
wherein a subject whose blood, serum, and/or plasma levels of PON1 enzymatic activity are low relative to a control or baseline value is at risk of experiencing a major adverse cardiac event within 3 years of the assessment.

2. The method of claim 1, wherein the PON1 enzymatic activity is determined by measuring arylesterase activity in the test subject's blood, serum, and/or plasma sample.

3. The method of claim 1, wherein the PON1 enzymatic activity is determined by measuring paraoxonase activity in the subject's blood, serum, and/or plasma sample

4. The method of claim 1, further comprising comparing the levels of PON enzymatic activity in the subject's blood, serum, and/or plasma sample with a control value based on PON1 enzymatic levels in blood, serum, and/or plasma samples from the general population.

5. The method of claim 1, further comprising comparing the levels of PON enzymatic activity in the subject's blood, serum, and/or plasma sample with a control value based on PON1 enzymatic levels in blood, serum, and/or plasma samples from a population of apparently healthy control subjects.

6. The method of claim 1, further comprising comparing the levels of PON1 enzymatic activity in the subject's blood, serum, and/or plasma sample with a baseline valued derived by measuring PON1 enzymatic levels in blood, serum, and/or plasma samples from the subject at an earlier time.

7. The method of claim 1, which further comprises comparing the levels of PON1 enzymatic activity in the subject's blood, serum, and/or plasma sample with a control value based on PON1 enzymatic levels in blood, serum, and/or plasma samples from a population of subjects who exhibit no symptoms or clinical evidence of cardiovascular disease within a predetermined period of time after providing said sample.

8. A method for assessing the risk of a subject experiencing a subsequent major adverse cardiac event within 3 years of the assessment, wherein the subject has experienced a first major adverse cardiac event comprising:
determining the levels of paraoxonase 1 (PON1) enzymatic activity in a blood, serum, and/or plasma sample from the subject,
wherein a subject whose blood, serum, and/or plasma levels of PON1 enzymatic activity are low relative to a control or baseline value is at risk of experiencing a subsequent major adverse cardiac event within 3 years of the assessment.

9. The method of claim 8 wherein the PON1 enzymatic activity in the subject's blood, serum, or plasma is determined by measuring paraoxonase activity or arylesterase activity in the subject's blood, serum, or plasma.

## Patentansprüche

1. Verfahren zum Bestimmen des Risikos eines Individuums, innerhalb von drei Jahren ab der Bestimmung ein schwerwiegendes, unerwünschtes kardiales Ereignis zu erleiden, wobei das Individuum keine Vorgeschichte von kardiovaskulärer Erkrankung und/oder weniger als 50 % Stenose aller großen Koronargefäße aufweist, wobei das Verfahren Folgendes umfasst:
Bestimmen des Ausmaßes von Paraoxonase-1- (PON1-) Enzymaktivität in einer Blut-, Serum- und/oder Plasmaprobe des Individuums;
wobei ein Individuum, dessen Blut-, Serum- und/oder Plasmaausmaß von PON1-Enzymaktivität bezogen auf einen Kontroll- oder Bezugswert niedrig ist, das Risiko aufweist, innerhalb von drei Jahren ab der Bestimmung ein schwerwiegendes, unerwünschtes kardiales Ereignis zu erleiden.

2. Verfahren nach Anspruch 1, wobei die PON1-Enzymaktivität durch Messen der Arylesteraseaktivität in der Blut-, Serum- und/oder Plasmaprobe der Versuchsperson bestimmt wird.

3. Verfahren nach Anspruch 1, worin die PON1-Enzymaktivität durch Messen der Paraoxonaseaktivität in der Blut-, Serum- und/oder Plasmaprobe des Individuums bestimmt wird.

4. Verfahren nach Anspruch 1, weiters umfassend das Vergleichen der Ausmaße von PON1-Enzymaktivität in der Blut-, Serum- und/oder Plasmaprobe des Individuums mit einem Kontrollwert basierend auf PON1-Enzymausmaßen in Blut-, Serum- und/oder Plasmaproben der Allgemeinpopulation.

5. Verfahren nach Anspruch 1, weiters umfassend das Vergleichen der Ausmaße von PON1-Enzymaktivität in der Blut-, Serum- und/oder Plasmaprobe des Individuums mit einem Kontrollwert basierend auf PON1-Enzymausmaßen in Blut-, Serumund/oder Plasmaproben einer Population von offensichtlich gesunden Kontrollindividuen.

6. Verfahren nach Anspruch 1, weiters umfassend das Vergleichen der Ausmaße von PON1-Enzymaktivität in der Blut-, Serum- und/oder Plasmaprobe des Individuums mit einem Bezugswert, der durch Messen der PON1-Enzymausmaße in Blut-, Serum- und/oder Plasmaproben des Individuums zu einem früheren Zeitpunkt abgeleitet ist.

7. Verfahren nach Anspruch 1, das weiters das Vergleichen der Ausmaße von PON1-Enzymaktivität in der Blut-, Serum- und/oder Plasmaprobe des Individuums mit einem Kontrollwert basierend auf PON1-Enzymaktivität in Blut-, Serum- und/oder Plasmaproben einer Population von Individuen umfasst, die keine Symptome oder keinen klinischen Nachweis kardiovaskulärer Erkrankung innerhalb einer vorgegebenen Zeitdauer nach dem Bereitstellen der Probe aufweisen.

8. Verfahren zum Bestimmen des Risikos eines Individuums, innerhalb von drei Jahren ab der Bestimmung ein schwerwiegendes, unerwünschtes kardiales Folgeereignis zu erleiden, wobei das Individuum ein schwerwiegendes, unerwünschtes kardiales Erstereignis erlitten hat, Folgendes umfassend:
Bestimmen der Ausmaße von Paraoxonase-1- (PON1-) Enzymaktivität in einer Blut-, Serum- und/oder Plasmaprobe des Individuums,
wobei ein Individuum, dessen Blut-, Serum- und/oder Plasmaausmaße von PON1-Enzymaktivität bezogen auf einen Kontroll- oder Bezugswert niedrig sind, das Risiko aufweist, ein schwerwiegendes, unerwünschtes kardiales Folgeereignis innerhalb von drei Jahren ab der Bestimmung zu erleiden.

9. Verfahren nach Anspruch 8, wobei die PON1-Enzymaktivität in dem Blut, Serum oder Plasma des Individuums durch Messen der Paraoxonaseaktivität oder Arylesteraseaktivität in dem Blut, Serum oder Plasma des Individuums bestimmt wird.

## Revendications

1. Procédé pour évaluer le risque chez un sujet de survenue d'un événement cardiaque indésirable majeur dans les 3 ans suivant l'évaluation, où le sujet n'a aucun antécédent de maladie cardiovasculaire et/ou présente une sténose de tous les principaux vaisseaux coronaires inférieure à 50 %, le procédé comprenant :
la détermination des niveaux d'activité enzymatique de paraoxonase 1 (PON1) dans un échantillon de sang, de sérum et/ou de plasma du sujet ;
où un sujet dont les niveaux d'activité enzymatique de PON1 dans le sang, le sérum, et/ou le plasma sont faibles par rapport à une valeur témoin ou initiale est exposé à un risque de survenue d'un événement cardiaque indésirable majeur dans les 3 ans suivant l'évaluation.

2. Procédé selon la revendication 1, dans lequel l'activité enzymatique de PON1 est déterminée par mesure de l'activité d'arylestérase dans l'échantillon de sang, de sérum, et/ou de plasma du sujet testé.

3. Procédé selon la revendication 1, dans lequel l'activité enzymatique de PON1 est déterminée par mesure de l'activité de paraoxonase dans l'échantillon de sang, de sérum, et/ou de plasma du sujet.

4. Procédé selon la revendication 1, comprenant en outre la comparaison des niveaux d'activité enzymatique de PON1 dans l'échantillon de sang, de sérum, et/ou de plasma du sujet à une valeur témoin basée sur les niveaux enzymatiques de PON1 dans des échantillons de sang, de sérum, et/ou de plasma de la population générale.

5. Procédé selon la revendication 1, comprenant en outre la comparaison des niveaux d'activité enzymatique de PON1 dans l'échantillon de sang, de sérum, et/ou de plasma du sujet à une valeur témoin basée sur les niveaux enzymatiques de PON1 dans des échantillons de sang, de sérum, et/ou de plasma d'une population de sujets témoins apparemment sains.

6. Procédé selon la revendication 1, comprenant en outre la comparaison des niveaux d'activité enzymatique de PON1 dans l'échantillon de sang, de sérum, et/ou de plasma du sujet à une valeur initiale obtenue en mesurant les niveaux enzymatiques de PON1 dans des échantillons de sang, de sérum, et/ou de plasma du sujet à un moment antérieur.

7. Procédé selon la revendication 1, qui comprend en outre la comparaison des niveaux d'activité enzymatique de PON1 dans l'échantillon de sang, de sérum, et/ou de plasma du sujet à une valeur témoin basée sur les niveaux enzymatiques de PON1 dans des échantillons de sang, de sérum, et/ou de plasma d'une population de sujets qui ne présentent aucun symptôme ni signe clinique de maladie cardiovasculaire au cours d'une période prédéterminée après avoir fourni ledit échantillon.

8. Procédé pour évaluer le risque chez un sujet de survenue d'un nouvel événement cardiaque indésirable majeur dans les 3 ans suivant l'évaluation, où le sujet a déjà connu un premier événement cardiaque indésirable majeur comprenant :
la détermination des niveaux d'activité enzymatique de paraoxonase 1 (PON1) dans un échantillon de sang, de sérum et/ou de plasma du sujet,
où un sujet dont les niveaux d'activité enzymatique de PON1 dans le sang, le sérum, et/ou le plasma sont faibles par rapport à une valeur témoin ou initiale est exposé à un risque de survenue d'un nouvel événement cardiaque indésirable majeur dans les 3 ans suivant l'évaluation.

9. Procédé selon la revendication 8 dans lequel l'activité enzymatique de PON1 dans le sang, le sérum ou le plasma du sujet est déterminé par mesure de l'activité de paraoxonase ou de l'activité d'arylestérase dans le sang, le sérum ou le plasma du sujet.
